**Europäisches Patentamt**

**European Patent Office**

**Office européen des brevets**

(19)

(11) Publication number : **0 328 685 B1**

## (12) EUROPEAN PATENT SPECIFICATION

(45) Date of publication of patent specification :
**13.05.92 Bulletin 92/20**

(51) Int. Cl.⁵ : **A61M 13/00**

(21) Application number : **88907366.4**

(22) Date of filing : **17.08.88**

(86) International application number :
**PCT/JP88/00811**

(87) International publication number :
**WO 89/01348 23.02.89 Gazette 89/05**

(54) **POWDERY MEDICINE APPLICATOR DEVICE.**

(30) Priority : **20.08.87 JP 205107/87**

(43) Date of publication of application :
**23.08.89 Bulletin 89/34**

(45) Publication of the grant of the patent :
**13.05.92 Bulletin 92/20**

(84) Designated Contracting States :
**AT BE CH DE FR GB IT LI NL SE**

(56) References cited :
**CH-A- 532 513**
**DE-A- 837 447**
**US-A- 1 671 011**
**US-A- 2 534 636**
**US-A- 2 946 332**

(73) Proprietor : **TEIJIN LIMITED**
**6-7, Minamihonmachi 1-chome Chuo-ku**
**Osaka-shi Osaka 541 (JP)**

(72) Inventor : **IKURA, Hiroshi**
**3-15-1-5-405 Suzaku**
**Nara-shi Nara (JP)**
Inventor : **SUZUKI, Yoshiki**
**5-20-2, Tamadaira Hino-shi**
**Tokyo 191 (JP)**

(74) Representative : **Cookson, Barbara Elizabeth**
**et al**
**WITHERS & ROGERS 4 Dyer's Buildings**
**Holborn**
**London EC1N 2JT (GB)**

Jouve, 18, rue Saint-Denis, 75001 PARIS

## Description

### TECHNICAL FIELD

The present invention relates to an applicator device for powdery medicines, according to the first part of claim 1. Such a device is known from the US-A-2534636.

### BACKGROUND ART

For patients suffering from a nose allergy, asthma, flat herpes, wide-range inflammatory stomatitis or a like disease, a curative method has been adopted in which a powdery medicine is applied into the nasal cavity or oral cavity.

In this curative method, a capsule filled with a powdery medicine is used as the powdery medicine applicator, and a device is known comprising an element for forming at least two holes in this powdery medicine-filled capsule and an element for feeding an air current in the perforated capsule to cause the powdery medicine to float in the air current.

For example, Japanese Unexamined Patent Publication (Kokai) No. 59-34267 discloses a device comprising (1) means for introducing an air current into a capsule for inhalation by a patient, (2) a housing for guiding a powdery medicine contained in the capsule into the nose or oral cavity of the patient, (3) a cap for the housing, and (4) a needle for perforating the capsule, which is attached to the interior of the cap so that the length of the needle is shorter than the length of the cap.

Although the size of this device is sufficiently compact, the device causes inconvenience to a user because the device must be always carried together with powdery medicine-filled capsules.

Moreover, for an aged or juvenile patient, the operation of attaching a capsule to the device, and the subsequent operations for completion of spraying, are complicated and cumbersome, and the user is likely to forget to return the parts of the device to the original positions.

Another problem of the conventional technique is that, since a pressing element such as a rubber ball is used in the conventional device, a reduction of the size is restricted. Moreover, if a desiccant is placed in a capsule for preventing a powdery medicine contained in the capsule from being destabilized by an absorption of moisture, water in the capsule is migrated in the desiccant, and a problem such as cracking occurs when the capsule is perforated by a needle with the result that an appropriate perforation cannot be obtained.

### DISCLOSURE OF THE INVENTION

Accordingly, an object of the present invention is to solve the above-mentioned problems of the conventional powdery medicine applicator device concerning the handiness when carried, ease of operation, and expedition of the application, and to provide a device which contains a powdery medicine in an amount corresponding to one dose or several doses, and which can apply the powdery medicine to the nasal cavity or other affected part by a feeding or suction of air only by forming a powdery medicine-spraying hole or this spraying hole and an air-introducing hole at the time of application, and which can be discarded after application or can be used again.

Another object of the present invention is to provide a powdery medicine applicator device which contains a powdery medicine in an amount corresponding to one dose or several doses, which can apply the powdery medicine to the nasal cavity or other affected part only by forming a powdery medicine-spraying hole at the time of application, and can be discarded after application or can be used again, and in which a capsule is easily perforated without trouble even if a desiccant is included for stabilizing the powdery medicine.

The solution to the problems is disclosed in the record part of claim 1.

### BRIEF DESCRIPTION OF THE DRAWINGS

Figures 1 to 7 show devices each having a powdery medicine impermeable and air permeable wall at least at the bottom of a medicine-holding portion, wherein:

Figure 1 is a sectional view showing a device in which an opening for spraying a powdery medicine is formed by wrenching off a knob connected to the head of a medicine-holding portion;

Fig. 2 is a sectional view showing a device and a cap, in which an opening is formed by putting the cap having a projection at the center on the inner side on the head of a medicine-holding portion;

Fig. 3 is a sectional view illustrating a device in which a powdery medicine-spraying opening is formed by wrenching off a knob connected to the head of a medicine-holding portion;

Fig. 4 is a sectional view showing a device and a cap, in which an opening is formed by putting the cap having a projection at the center on the inner side on the head of a medicine-holding portion;

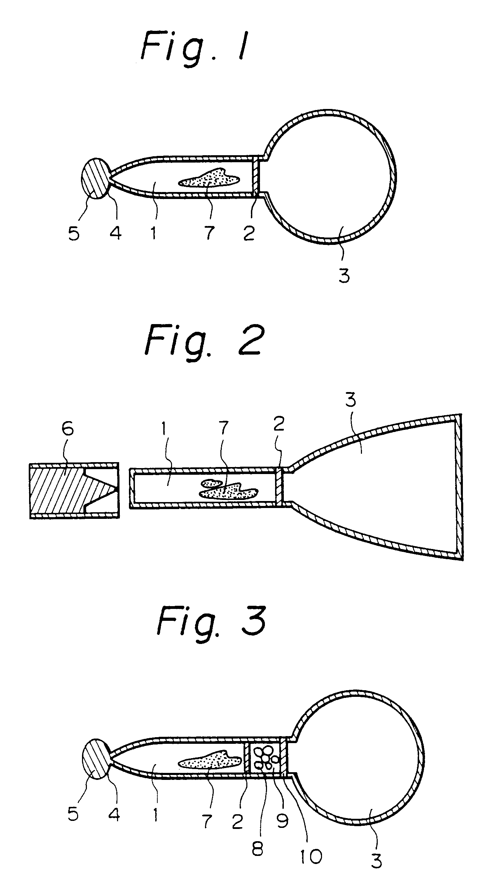

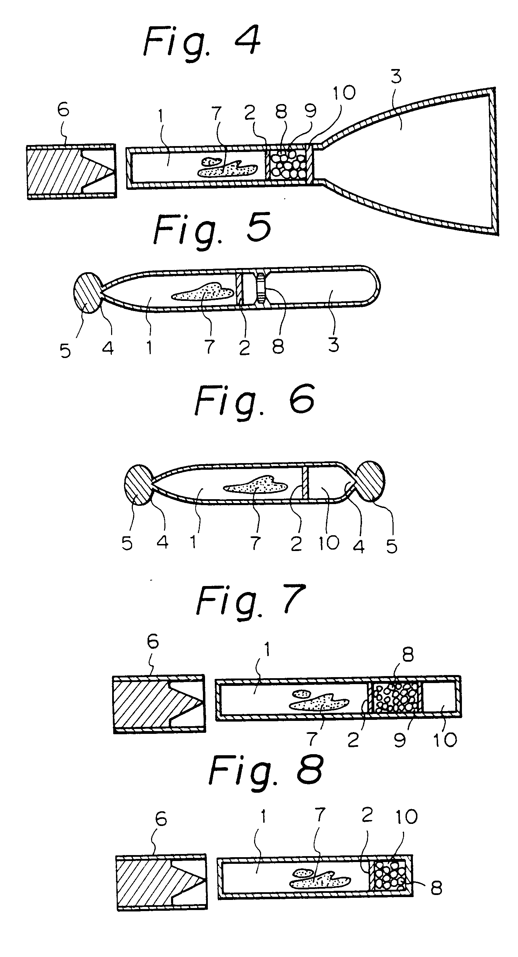

Figs. 3 and 4 show a desiccant held between partition walls or between a partition wall and a net;

Fig. 5 is a sectional view showing a device in which an opening is formed in the same manner as in the device shown in Fig. 1, and a desiccant is fixed on the inner circumference of an air reservoir (desiccant-holding portion) ;

Fig. 6 is a sectional view illustrating a device in which a powdery medicine-inhaling opening is formed by wrenching off a knob connected to the head of a medicine-holding portion and an air-introducing opening is formed by wrenching off a knob connected to the bottom of an air-introducing portion; and

Fig. 7 is a sectional view illustrating a device and a cap, in which an opening is formed by putting the cap having a projection at the center on the inner side on the head of a medicine-holding portion and the bottom of an air-introducing portion, and a desiccant is contained in the air-introducing portion.

## BEST MODE FOR CARRYING OUT THE INVENTION

In accordance with the first embodiment of the present invention, the air-introducing means of the powdery medicine applicator device is an air reservoir formed of a flexible material, and the bottom of the medicine-holding portion is connected to the air reservoir through a powdery medicine-impermeable and air-permeable partition wall.

In accordance with the second embodiment of the present invention, the air-introducing means of the powdery medicine applicator device is an air reservoir formed of a flexible material, and the powdery medicine applicator device comprises the above-mentioned medicine-holding portion, this device further comprises a desiccant-holding portion, and the bottom of the medicine-holding portion is connected to the air reservoir through a powdery medicine-impermeable and air-permeable partition wall and the desiccant-holding portion, or this device contains a desiccant in the air reservoir.

In accordance with the third embodiment of the present invention, the air-introducing means of the powdery medicine applicator device is an air-introducing portion having an air-introducing hole formed on the bottom thereof, and the bottom of the medicine-holding portion is connected to the air-introducing portion through a powdery medicine-impermeable and air-permeable partition wall.

In accordance with the fourth embodiment of the present invention, the air-introducing means of the powdery medicine applicator device is an air-introducing portion having an air-introducing hole formed on the bottom thereof and further having a desiccant-holding portion, and the bottom of the medicine-holding portion is connected to the air-introducing portion through a powdery medicine-impermeable and air-permeable partition wall and the desiccant-holding portion, or a desiccant is contained in the air-introducing portion.

In the powdery medicine applicator device of the present invention, a powdery medicine is sealed in the interior of the medicine-holding portion, and before application, the medicine-holding portion is kept in the sealed state. The shape of the medicine-holding portion is not particularly critical, so far as the shape is adaptable to the nasal cavity or other affected part to which the powdery medicine is applied and spraying or inhalation of the sealed powdery medicine is not inhibited by the medicine-holding portion. For example, a cylindrical shape can be used.

Note, 5 to 500 mg of the powdery medicine is mechanically filled in the medicine-holding portion in advance.

In the device of the present invention, the medicine-holding portion is constructed so that an opening for spraying or inhaling the powdery medicine will be formed at the head of the medicine-holding portion. As the means for forming this opening, there can be mentioned, for example, a method in which a knob having a slender neck connected to the medicine-holding portion is wrenched off to expose a small-diameter open end leading to the head of the medicine-holding portion, whereby an opening is formed, and a method in which a cap having a projection at the center on the inner side is put on the head of the medicine-holding portion to form an opening.

Preferably, the thickness of the head of the medicine-holding portion is small, as the hole can be then formed easily and simply.

In general, this medicine-holding portion is preferably composed of a polymer such as polyethylene, polystyrene, polypropylene, a styrene/acrylonitrile copolymer or an acrylonitrile/butadiene/styrene copolymer.

The size of the medicine-holding portion is appropriately determined according to the medicine-applying portion, such as nasal cavity, the oral cavity, the pharynx, the lung or other affected part.

In the first and second embodiments of the present invention, the air reservoir is an element for compressing air necessary for spraying the powdery medicine and is composed of a flexible material, for example, an elastic plastic material such as polyethylene or polypropylene, or a natural or synthetic rubber.

The medicine-holding portion can be composed of the same material as that of the air reservoir.

Preferably, the size of the air reservoir is such that, by depressing or pressing the air reservoir 1 to 10 times, air is discharged in an amount sufficient to discharge substantially all of the powdery medicine sealed in the medicine-holding portion. At the time of application, air introduced into the air reservoir is limited to that coming from the opening formed at the head of the medicine-holding portion.

In the present invention, the bottom of the medicine-holding portion is connected to the air reservoir through a powdery medicine-impermeable and air-permeable partition wall (further, through a desiccant-holding portion in the second embodiment).

A sieving net having a mesh size of 5 to 37 $\mu$m,

preferably 5 to 10 µm, or a membrane filter having a pore size of 5 to 75 µm, preferably 5 to 25 µm, is used as the partition wall in the present invention.

For example, a sieving net formed of nylon or stainless steel or a membrane filter formed of polypropylene, cotton, rayon or glass fiber can be used. Of course, the constituting material is not limited to those mentioned above, so long as the mesh size or pore size is within the above-mentioned range.

In the first and second embodiments of the present invention, it is generally considered that, when air is caused to flow into the air reservoir from the medicine-holding portion after formation of the opening, the powdery medicine sealed in the medicine-holding portion may flow backward through the sieving net or membrane filter, but where at least about 90% by weight of particles of the powdery medicine have an effective particle size of about 20 to about 200 micrometres, a backward flow of the powdery medicine will not occur.

In the second embodiment of the present invention, preferably the desiccant in the desiccant-holding portion is held or fixed to the desiccant-holding portion by some means. For example, the air reservoir side of the desiccant-holding portion may be constructed by a powdery medicine-impermeable and air-permeable partition wall or a net having a mesh size such that it will not allow the desiccant to fall into the air reservoir. Furthermore, a ring-shaped desiccant may be fixed to the inner wall of the desiccant-holding portion.

As the partition wall or net constructing the air reservoir side of the desiccant-holding portion in the present invention, there can be used a sieving net having a mesh size of 5 µm to 1 mm, preferably 5 to 200 µm, and a membrane filter having a pore size of 5 to 75 µm, preferably 5 to 25 µm.

A sieving net formed of nylon or stainless steel or a membrane filter composed of polypropylene, cotton, rayon or glass fiber can be used, but the constituting material is not particularly critical, so long as the mesh size or pore size is within the above-mentioned range.

As the desiccant to be used in the present invention, there can be mentioned plate-shaped or granular silica gel or calcium chloride, but the kind of the desiccant is not particularly critical, so long as the safety is high and the drying capacity is satisfactory.

In the present invention, the effect of the stabilizing the powdery medicine of the present invention can be enhanced by using an oxygen absorber in addition to the desiccant.

In the third embodiment of the present invention, the air-introducing portion is constructed so that an air-introducing opening is formed on the bottom of the air-introducing portion. As the means for forming this opening, a method can be adopted in which a knob having a slender neck connected to the bottom of the air-introducing portion is wrenched off to expose a small-diameter open end connected to the rear end of the air-introducing portion, whereby an opening is formed, and a method in which a cap having a projection at the center on the inner side is put on the bottom of the air-introducing portion to form an opening.

In the fourth embodiment of the present invention, a desiccant can be held in a desiccant-holding portion or the air-introducing portion in the same manner as described above. By thus holding the desiccant, even if there is a risk of degradation of the stability of the powdery medicine by an absorption of moisture, the stability of the medicine can be maintained at a high level. Furthermore, by holding an oxygen absorber in addition to the desiccant, the effect of stabilizing the medicine can be further enhanced.

The powdery medicine applicator device according to the third embodiment of the present invention is advantageous in that, since the medicine is contained in the device per se and a pressing element such as a rubber ball is not used, the size can be reduced and thus the handiness improved.

The powdery medicine applicator device of the present invention is characterized, first in that, since the powdery medicine is contained in the device per se, the device is easy to carry. In the second place, the device of the present invention is characterized in that, by containing the powdery medicine in an amount corresponding to one or several doses, the applicator can be discarded after application or can be used again. The device of the present invention is further characterized in that the number of constituent elements is very small and the device can be fabricated at a very low cost.

According to the second embodiment of the present invention, since the device is designed so that a desiccant can be held in the device, the risk of reduction of the stability of the powdery medicine sealed in the medicine-holding portion by an absorption of moisture can be eliminated and the stability of the powdery medicine can be maintained at a high level.

## EXAMPLES

The present invention will now be described in detail with reference to the examples illustrated in the accompanying drawings, which by no means limit the scope of the invention.

## Example 1

Figure 1 illustrates the state where a powdery medicine 7 is filled in a medicine-holding portion 1 having a capacity capable of containing a powdery medicine therein. At the time of application, a knob 5 formed on the head end of the medicine-holding portion is wrenched off to expose a small-diameter open end 4 and form an opening. Then, by depressing or pressing air reservoir 3, air in the air reservoir 3 is caused to flow into the medicine-holding portion from the air reservoir through a partition wall 2 and the powdery medicine sealed in the medicine-holding portion is sprayed to the affected part. Air necessary for spraying at the subsequent application is introduced into the air reservoir by a backward flow from the opening at the head of the medicine-holding portion. At this point, a backward flow of the unsprayed powdery medicine is prevented by the partition wall, and thus is not introduced into the air reservoir. Therefore, loss of the powdery medicine can be prevented and substantially all of the powdery medicine can be sprayed to the affected part.

Figure 2 illustrates an embodiment in which a cap 6 having a projection at the center on the inner side is used for formation of an opening at the head of the medicine-holding portion 1, and an opening is formed by putting this cap on the head of the medicine-holding portion 1.

The subsequent operations are the same as in the embodiment shown in Fig. 1.

In this embodiment, if the powdery medicine is not completely sprayed but a part of the medicine is left, by sealing the device with the cap and storing the device in this state, the device can be used again.

## Example 2

Figure 3 illustrates the state where a powdery medicine 7 is filled in a medicine-holding portion 1 having a capacity capable of containing a powdery medicine therein, and shows a desiccant held between partition walls. At the time of application, a knob 5 formed on the top end of the medicine-holding portion is wrenched off to expose a small-diameter open end and form an opening. Then, by depressing or pressing an air-pressing air reservoir 3, air in the air reservoir is caused to flow into the medicine-holding portion from the air reservoir through a desiccant-holding portion 9 and a partition wall 2, and the powdery medicine sealed in the medicine-holding portion is sprayed on the affected part. Air necessary for spraying at the subsequent spraying is introduced into the air reservoir by a backward flow from the opening of the medicine-holding portion. At this point, a backward flow of the unsprayed powdery medicine is prevented by the partition wall, and thus is not introduced into the desiccant-holding portion or the air reservoir. Therefore, loss of the powdery medicine can be prevented and substantially all of the powdery medicine can be sprayed to the affected part.

Figure 4 illustrates an embodiment in which a cap 6 having a projection at the center on the inner side is used for forming an opening at the head of the medicine-holding portion 1, and an opening is formed by putting this cap on the head of the medicine-holding portion 1. Other operations are the same as in the embodiment shown in Fig. 3.

In this embodiment, if the powdery medicine is not completely sprayed but a part of the medicine is left, by sealing the device with the cap and storing the device in this state, the device can be used again.

Figure 5 illustrates an embodiment in which a desiccant is fixed in the annular form along the inner circumference of the air reservoir. In this embodiment, the air reservoir also acts as a desiccant-holding portion.

## Example 3

Figure 6 illustrates the state where a powdery medicine 7 is filled in a medicine-holding portion 1 having a capacity capable of containing a powdery medicine therein. At the time of application, a knob 5 formed on the head of the medicine-holding portion 1 is wrenched off to expose a small-diameter open end 4 and form an opening, and another knob 5 formed on the bottom of an air-introducing portion 10 is wrenched off to form an air-introducing opening. Then, the small-diameter open end at the head of the medicine-holding portion 1 is fitted to the nasal cavity or oral cavity, and by the inhaling operation of the breathing movement, the powdery medicine sealed in the medicine-holding portion is inhaled in the affected part. At the subsequent inhalation, the powdery medicine is inhaled, and even if the small-diameter open end remains fitted to the nasal cavity or oral cavity, the uninhaled powdery medicine is not introduced in the air-introducing portion 10 from the bottom of the medicine-holding portion 1 by the air breathed out from the nasal cavity or oral cavity. Therefore, any loss of the powdery medicine can be prevented and substantially all of the powdery medicine can be inhaled.

Figures 7 and 8 illustrates an embodiment in which a cap 6 having a projection at the center on the inner side is used for forming an opening on the head of the medicine-holding portion 1 and the bottom of the air-introducing portion 10, and an opening is formed by putting this cap on the head of the medicine-holding portion 1 or the bottom of the air-introducing portion 10. A desiccant 8 may be contained in a desiccant-holding portion 9 (Fig. 7) or in the air-introducing portion 10 (Fig. 8). The subsequent operations are the same as in the embodiment shown in Fig. 6.

## Claims

1. An applicator device for powdery medicines, the device comprising a medicine-holding portion (1) containing powdery medicine (7), means (3, 10) for introducing air at a first end thereof, and means (4) at a second end thereof for adminestering powdery medicine to an affected part of the body, the medecine-holding (1) portion being connected to the air-introducing means (3, 10) via a partition wall (2) which is impermeable to powdery medicine but permeable to air, characterized in that the device comprises a container which is closed overall whereby the powdery medicine is sealed therein and in which the powdery medicine administration means is capable of having an opening formed therein for use.

2. A powdery medicine applicator device as claimed in Claim 1, wherein the air-introducing means comprises an air reservoir (3) composed of a flexible material.

3. A powdery medicine applicator device as claimed in Claim 2, wherein the opening for administering the powdery medicine is formed by wrenching off a knob (5) connected to the head of the medicine-holding portion.

4. A powdery medicine applicator device as claimed in Claim 2, wherein the opening for administering the powdery medicine is formed by putting a cap (6) having a projection at the center of the inner side on the head of the medicine-holding portion.

5. A powdery medicine applicator device as claimed in Claim 2, which further comprises a desiccant-holding portion (9), wherein the first end of the medecine-holding portion (1) is connected to the air reservoir (3) through a powdery medicine-impermeable and air-permeable partition wall and the desiccant-holding portion, or wherein a desiccant is contained in the air reservoir.

6. A powdery medicine applicator device as claimed in Claim 1, wherein the air-introducing means is a portion (10) constructed so that an air-introducing opening may be formed at the first end.

7. A powdery medicine applicator device as claimed in Claim 6, wherein te opening for administering the powdery medicine and the air-introducing opening are formed by wrenching off knobs (5) formed on the head of the medicine-holding portion and the bottom of the air-introducing portion, respectively.

8. A powdery medicine applicator device as claimed in Claim 6, wherein the opening for administering the powdery medicine and the air-introducing opening are formed by putting a cap (6) having a projection at the center of the inner side on the head of the medicine-holding portion and the first end of the air-introducing portion, respectively.

9. A powdery medicine applicator device as claimed in Claim 6, which further comprises a desiccant-holding portion (9), wherein the first end of the medicine-holding portion (1) is connected to the air reservoir through a powdery medicine-impermeable and air-permeable partition wall and the desiccant-holding portion, or wherein a desiccant is contained in the air-introducing portion.

**Patentansprüche**

1. Applikator für pulverförmige Arzneimittel, welcher umfaßt: einen Arzneimittelaufnahmeteil (1), welcher das pulverförmige Arzneimittel (7) enthält, Einrichtungen (3, 10) zum Einführen von Luft an einem ersten Ende desselben und Einrichtungen (4) an einem zweiten Ende desselben zum Verabreichen des pulverförmigen Arzneimittels an einen betroffenen Teil des Körpers, wobei der Arzneimittelaufnahmeteil (1) mit den Luftzuführeinrichtungen (3, 10) über eine Trennwand (2) verbunden ist, welche für das pulverförmige Arzneimittel undurchlässig, jedoch für Luft durchlässig ist, **dadurch gekennzeichnet**, daß der Applikator einen Behälter umfaßt, der insgesamt geschlossen ist, so daß das pulverförmige Arzneimittel darin dichtend eingeschlossen ist, und in dem zur Benutzung der Arzneimittel-Verabreichungseinrichtungen eine Öffnung erzeugbar ist.

2. Applikator für pulverförmige Arzneimittel nach Anspruch 1, bei dem die Luftzuführeinrichtungen ein aus einem flexiblen Material zusammengesetztes Luftreservoir (3) umfassen.

3. Applikator für pulverförmige Arzneimittel nach Anspruch 2, bei dem die Öffnung zum Verabreichen des pulverförmigen Arzneimittels dadurch gebildet wird, daß ein mit dem Kopf des Arzneimittelaufnahmeteils verbundener Knopf (5) abgedreht wird.

4. Applikator für pulverförmige Arzneimittel nach Anspruch 2, bei dem die Öffnung zum Verabreichen des pulverförmigen Arzneimittels dadurch hergestellt wird, daß eine Kappe (6) mit einem Vorsprung in der Mitte ihrer Innenseite auf den Kopf des Arzneimittelaufnahmeteils aufgesetzt wird.

5. Applikator für pulverförmige Arzneimittel nach Anspruch 2, welcher ferner einen Trocknungsmittelaufnahmeteil (9) umfaßt, wobei das erste Ende des Arzneimittelaufnahmeteils (1) mit dem Luftreservoir (3) über eine für das pulverförmige Arzneimittel unterdurchlässige, jedoch für Luft durchlässige Trennwand und den Trocknungsmittelaufnahmeteil verbunden ist, oder wobei ein Trocknungsmittel in dem Luftreservoir enthalten ist.

6. Applikator für pulverförmige Arzneimittel nach Anspruch 1, bei dem die Luftzuführeinrichtungen ein Teil (10) sind, welches so konstruiert ist, daß an dem ersten Ende eine Luftzuführöffnung erzeugt werden kann.

7. Applikator für pulverförmige Arzneimittel nach Anspruch 6, bei dem die Öffnung zum Verabreichen des pulverförmigen Arzneimittels und die Luftzuführöffnung durch Abdrehen von Knöpfen (5) gebildet werden, die am Kopf des Arzneimittelaufnahmeteils bzw. am Boden des Luftzuführteils vorgesehen sind.

8. Applikator für pulverförmige Arzneimittel nach Anspruch 6, bei dem die Öffnung zum Verabreichen des pulverförmigen Arzneimittels und die Luftzuführöffnung hergestellt werden, indem eine Kappe (6) mit einem Vorsprung in der Mitte ihrer Innenseite auf den Kopf des Arzneimittelaufnahmeteils bzw. auf das erste Ende des Luftzuführteils aufgesetzt wird.

9. Applikator für pulverförmige Arzneimittel nach Anspruch 6, welcher ferner einen Trocknungsmittelhalteteil (9) umfaßt, wobei das erste Ende des Arzneimittelaufnahmeteils (1) mit dem Luftreservoir über eine für pulverförmige Arzneimittel undurchlässige, jedoch für Luft durchlässige Trennwand und den Trocknungsmittelaufnahmeteil verbunden ist oder wobei das Trocknungsmittel in dem Luftzuführteil enthalten ist.

## Revendications

1. Dispositif applicateur pour médicaments en poudre, le dispositif comprenant une partie contenant le médicament (1) contenant le médicament en poudre (7), des organes d'introduction de l'air (3, 10) à une première extrémité de celle-ci et un organe (4) à la seconde extrémité de celle-ci destiné à l'administration du médicament en poudre à une partie concernée de l'organisme, la partie contenant le médicament (1) étant reliée aux organes d'introduction de l'air (3, 10) par une cloison (2) qui est imperméable au médicament en poudre, mais perméable à l'air, caractérisé en ce que le dispositif comprend un récipient globalement fermé, le médicament en poudre y étant scellé, et en ce qu'on peut former un orifice dans l'organe d'administration du médicament en poudre en vue de son emploi.

2. Dispositif applicateur pour médicaments en poudre selon la revendication 1, dans lequel l'organe d'introduction de l'air comprend un réservoir à air (3) en une matière souple.

3. Dispositif applicateur pour médicaments en poudre selon la revendication 2, dans lequel l'orifice d'administration du médicament en poudre est formé par arrachement par torsion d'un bouton (5) relié à la tête de la partie contenant le médicament.

4. Dispositif applicateur pour médicaments en poudre selon la revendication 2, dans lequel l'orifice d'administration du médicament en poudre est formé par positionnement d'un capuchon (6) présentant sur sa face interne une partie centrale en saillie, sur la tête de la partie contenant le médicament.

5. Dispositif applicateur pour médicaments en poudre selon la revendication 2, comprenant en outre une partie contenant un déshydratant (9), dans lequel la première extrémité de la partie contenant le médicament (1) est reliée au réservoir à air (3) par une cloison imperméable au médicament en poudre et perméable à l'air et la partie contenant le déshydratant ou dans lequel un déshydratant est enfermé dans le réservoir à air.

6. Dispositif applicateur pour médicaments en poudre selon la revendication 1, dans lequel l'organe d'introduction de l'air est une partie (10) conçue de façon telle qu'on peut former un orifice d'introduction de l'air à la première extrémité.

7. Dispositif applicateur pour médicaments en poudre selon la revendication 6, dans lequel l'orifice d'administration du médicament en poudre et l'orifice d'introduction de l'air sont formés par arrachement par torsion de boutons (5) formés respectivement sur la tête de la partie contenant le médicament et le fond de la partie d'introduction de l'air.

8. Dispositif applicateur pour médicaments en poudre selon la revendication 6, dans lequel l'orifice d'administration du médicament en poudre et l'orifice d'introduction de l'air sont formés par positionnement d'un capuchon (6) présentant sur sa face interne une partie centrale en saillie respectivement sur la tête de la partie contenant le médicament et la première extrémité de la partie d'introduction de l'air.

9. Dispositif applicateur pour médicaments en poudre selon la revendication 6, comprenant en outre une partie contenant un déshydratant (9), dans lequel la première extrémité de la partie contenant le médicament (1) est reliée au réservoir à air par une cloison imperméable au médicament en poudre et perméable à l'air et par la partie contenant le médicament, ou dans lequel un déshydratant est enfermé dans la partie d'introduction de l'air.

# Fig. 1

# Fig. 2

# Fig. 3

# Fig. 4

# Fig. 5

# Fig. 6

# Fig. 7

# Fig. 8